# EUROPÄISCHE PATENTANMELDUNG

(11) **EP 0 955 040 A1**
(43) Veröffentlichungstag der Anmeldung: **10.11.1999**
(21) Anmeldenummer: 99107080.6
(22) Anmeldetag: 12.04.1999
(51) Int. Cl.: A61K 9/16, A61K 9/20, A61K 31/185

(54) **Arzneiform zur oralen Applikation von Mesna**

(30) Priorität: 27.04.1998 DE 19818804
(71) Anmelder: ASTA Medica Aktiengesellschaft, 01277 Dresden (DE)
(72) Erfinder: Rawert, Jürgen, Dr., 63755 Alzenau (DE); Sarlikiotis, Werner, Dr., 33613 Bielefeld (DE)

(57) **Zusammenfassung**

Die Erfindung betrifft Arzneiformen zur oralen Applikation in Form von Tabletten, Filmtabletten, Pellets oder Granulat enthaltend mindestens 88% Mesna, hergestellt durch Granulation mit bis zu 15% Wasser bezogen auf den eingesetzten Feststoffanteil sowie Tabletten, Filmtabletten, Pellets oder Granulat enthaltend mindestens 80% Mesna, hergestellt durch Direktverpressung oder Kompaktierung. ohne Verwendung organischer Lösungsmittel.

## Beschreibung

Mesna ist ein bekanntes Antidot, welches zur Prophylaxe der Urotoxizität von Oxaphosphorinen wie lfosphamid und Cyclosphosphamid eingesetzt wird. Neben parenteralen sind auch bereits orale Formulierungen bekannt.

So sind im US-Patent 5,503,845 orale Formulierungen in Form von Tabletten, Pellets, Kapseln mit einem Wirkstoffgehalt von bis zu > 85% Mesna in Verbindung mit einem Herstellungsverfahren der Feuchtgranulierung mit mehr als 30% Wasser, bezogen auf die Menge der eingesetzten Feststoffe beschrieben. Problematisch sind hier die durch die große Wassermengen zu erwartenden langen und unwirtschaftlichen Trockenzeiten. Außerdem führen hohe Wassermengen auch zu Wirkstoffinstabilität.

Ferner sind aus US-Patent 5, 358, 718 Brausetabletten mit 10-80% Mesna. bekannt. US- Patent 5, 262, 169 beschreibt Tabletten mit 10-80% Mesna. Bei beiden o.g. Patentschriften ist die beanspruchte Zusammensetzung mit einem alkoholischen Granulationsverfahren gekoppelt.

Der Einsatz von organischen Flüssigkeiten bei der Granulation ist jedoch als problematisch einzustufen, da diese Stoffe meistens umweltschädlich sind, und außerdem spezielle Einrichtungen zum Mitarbeiterschutz benötigt werden.

EP 0468245 beschreibt Mesnatabletten, die 10-80% Mesna enthalten, in Kombination mit diversen Hilfsstoffen. Auch wird hier ein Verfahren zur Herstellung von diesen Tabletten mittels Granulation im Beisein organischer Lösungsmittel beschrieben. Es gelten die gleichen Probleme wie bei den o.g US Patenten.

Es besteht somit die Aufgabe Mesna Tabletten mittels einer einfachen, wirtschaftlichen Herstellungsmethode zu fertigen und dies möglichst ohne den Einsatz von organischen Lösungsmitteln.

Da Mesna hochdosiert appliziert wird, ist es erforderlich, daß die oralen Mesna Formulierungen einen Wirkstoffanteil von über 80% aufweisen.

Überraschenderweise konnte die erwähnte Aufgabe gelöst werden indem Arzneiformen zur oralen Applikation in Form von Tabletten, Filmtabletten, Pellets oder Granulat enthaltend mindestens 88% Mesna, durch Granulation mit bis zu 15% Wasser bezogen auf den eingesetzten Feststoffanteil hergestellt werden bzw. Arzneiformen zur oralen Applikation in Form von Tabletten, Filmtabletten, Pellets oder Granulat enthaltend mindestens 80% Mesna durch Direktverpressung oder Kompaktierung.

Hervorzuheben ist, daß das Verfahren zur Herstellung der Arzneiformen zur oralen Applikation in Form von Tabletten, Filmtabletten, Pellets oder Granulat durch Granulation, Direktverpressung oder Kompaktierung ohne Verwendung organischer Lösungsmittel auskommt.

An Hand der nachfolgenden Ausführungbeispiele wird die Erfindung näher erläutert ohne sie jedoch dadurch einzuschränken.

### Beispiel 1

### Tabletten mit 100% Mesna

500 g Mesna werden gesiebt und mit 97 g Wasser (= 19,4% bezogen auf den Feststoff) befeuchtet. Anschließend wird granuliert, und bei 40° C auf Horden getrocknet. Das Granulat wird zu Tabletten verpresst.
- Gewicht:: 500 mg
- Bruchfestigkeit:: 70-80 N
- Zerfall:: <1,5 min.

### Beispiel 2

### Tabletten mit 88% Mesna

### Rein wäßrige Granulation mit 5,6% Wasser

2,7 g Maisstärke werden in 3,3 g Wasser gelöst und in 13,7 g Wasser aufgequollen.

300 g Mesna werden gesiebt und zusammen mit aufgequollener Maisstärke geknetet.

Die feuchte Masse wird granuliert und bei 40° C auf Horden getrocknet. Das getrocknete Granulat wird mit 27,0 g mikrokristalline Cellulose und 6,0 g Maisstärke vermischt. Anschließend werden 2,7 g Magnesiumstearat hinzugegeben und erneut gemischt.
- Gewicht:: 225,6 mg
- Bruchfestigkeit:: 100 N
- Zerfall:: <4 min.

### Beispiel 3

### Tabletten mit 81,6% Mesna

### Kompaktierung.

200 g Mesna werden zusammen mit 30,0 g Lactose und 10,0 g hochdisperses Siliciumdioxid gesiebt und vermischt. Anschließend werden 5,0 g Magnesiumstearat zugegeben und erneut gemischt. Die so hergestellte Masse wird zu Komprimaten verpresst. Die Komprimate werden zerkleinert und gesiebt. Die so entstandene Masse wird gemischt und zu Tabletten verarbeitet.
- Gewicht:: 245 mg
- Bruchfestigkeit:: 50 N
- Zerfall:: <3 min.

## Patentansprüche

1. Arzneiformen zur oralen Applikation in Form von Tabletten, Filmtabletten, Pellets oder Granulat enthaltend mindestens 88% Mesna, hergestellt durch Granulation mit bis zu 15% Wasser bezogen auf den eingesetzten Feststoffanteil.

2. Arzneiformen zur oralen Applikation in Form von Tabletten, Filmtabletten, Pellets oder Granulat enthaltend mindestens 80% Mesna, hergestellt durch Direktverpressung oder Kompaktierung.

3. Verfahren zur Herstellung der Arzneiformen zur oralen Applikation in Form von Tabletten, Filmtabletten, Pellets oder Granulat enthaltend mindestens 88% Mesna, nach Anspruch 1 durch Granulation mit bis zu 15% Wasser bezogen auf den eingesetzten Feststoffanteil ohne Verwendung organischer Lösungsmittel.

4. Verfahren zur Herstellung von Arzneiformen zur oralen Applikation in Form von Tabletten, Filmtabletten, Pellets oder Granulat enthaltend mindestens 80% Mesna, nach Anspruch 2 durch Direktverpressung oder Kompaktierung ohne Verwendung organischer Lösungsmittel.
